**Europäisches Patentamt**

(19) **European Patent Office**

**Office européen des brevets**

(11) Veröffentlichungsnummer: **0 023 557**
**B1**

(12) # EUROPÄISCHE PATENTSCHRIFT

(45) Veröffentlichungstag der Patentschrift:
**01.08.84**

(21) Anmeldenummer: **80103403.4**

(22) Anmeldetag: **18.06.80**

(51) Int. Cl.³: **C 07 C 76/02,** C 07 C 79/46,
C 07 C 103/34, C 07 C 103/38,
C 07 B 11/00

(54) **Verfahren zur Herstellung von substituierten Nitroarylverbindungen.**

(30) Priorität: 21.06.79 CH 5806/79
04.09.79 CH 7980/79
09.04.80 CH 2716/80
09.04.80 CH 2717/80

(43) Veröffentlichungstag der Anmeldung:
**11.02.81 Patentblatt 81/6**

(45) Bekanntmachung des Hinweises auf die Patenterteilung:
**01.08.84 Patentblatt 84/31**

(84) Benannte Vertragsstaaten:
**BE CH DE FR GB LI**

(56) Entgegenhaltungen:
**EP - A - 0 028 203**
**DD - A - 123 453**
**DE - A - 2 249 373**
**DE - A - 2 522 818**
**DE - C - 1 129 471**

**Chemical Abstracts Band 84, Nr. 15, 1976 Columbus,
Ohio, USA J.W. CHAPMAN et al. "Two phase nitration of
chlorobenzene in 79,8% sulfuric acid" Seite 501,
Abstract Nr. 104690a**

(73) Patentinhaber: **CIBA-GEIGY AG, Postfach,
CH-4002 Basel (CH)**

(72) Erfinder: **Rosner, Manfred, Dr., Römerweg 12,
CH-4132 Muttenz (CH)**

(74) Vertreter: **Berg, Wilhelm, Dr. et al, Dr. Berg, Dipl.-Ing.
Stapf, Dipl.-Ing. Schwabe, Dr. Dr. Sandmair
Mauerkircherstrasse 45, D-8000 München 80 (DE)**

## Beschreibung

Gegenstand der Erfindung ist ein Verfahren zur Herstellung von mindestens monosubstituierten Nitroarylverbindungen durch chargenweise Nitrierung von mindestens monosubstituierten Arylverbindungen.

Nitrierungsreaktionen und nitrierte monosubstituierte Arylverbindungen haben in der Technik erhebliche Bedeutung erlangt. Die Verfahren zur Nitrierung werden dabei in Abhängigkeit der jeweilig erforderlichen Reinheit der Produkte, in bezug auf Nitrierungsgrad und Isomerenreinheit, individuell und chargenweise gestaltet, denn oft ist die Reaktionstemperatur kritisch und darf bestimmte Werte nicht überschreiten, anderseits spielt auch die Konzentration der zu verwendenden Säure und das Reaktionsmedium eine entscheidende Rolle.

Aus der DE-PS 1 129 471 ist ein Verfahren zur Herstellung von 3,5-Dinitro-o-toluylsäure bekannt. Dabei wird rohe o-Toluylsäure gelöst in einem niederaliphatischen Chlorkohlenwasserstoff mit einem Gemisch aus Salpetersäure und Schwefelsäure dinitriert.

Ferner ist in der DD-PS 123 453 ein Verfahren zur Herstellung von Nitroderivaten aromatischer Verbindungen beschrieben, nach dem die aromatische Verbindung in Gegenwart eines substituierten Kohlenwasserstoffs mit Salpetersäure oder einem Nitrierungsmittel nitriert wird. Dabei wird auf die Verwendung von Mineralsäuren als Lösungs- oder Verdünnungsmittel vollständig verzichtet.

Im Zuge der Vereinfachung von Verfahrenstechnologien, in bezug auf einen universellen Einsatz von Apparaten und insbesondere auf ökologisch günstigere Arbeitsweise, besteht daher in der Technik ein echter Bedarf an Verfahren, nach denen mehrere Produkte nach ein und demselben Verfahren hergestellt werden können. Der vorliegenden Erfindung liegt daher die Aufgabe zugrunde, ein Verfahren zu entwickeln, das generell anwendbar ist und den Nachteil der jeweilig individuellen Verfahrensgestaltung bei der Nitrierung von mindestens monosubstituierten Arylverbindungen nicht aufweist.

Diese Aufgabe wird durch das erfindungsgemässe Verfahren gelöst. Es wurde gefunden, dass bei chargenweiser Durchführung der Nitrierung in einem Reaktionsmedium, das aus zwei Flüssigphasen besteht, wobei eine der Phasen eine organische Phase, bestehend aus einer organischen Flüssigkeit, die reaktionsinert ist, also an der Reaktion nicht teilnimmt, und in der das Nitrierungsprodukt in Gegenwart von konzentrierter Schwefelsäure praktisch unlöslich ist und die andere eine anorganische Phase darstellt, bestehend aus mindestens 80%iger Schwefelsäure bei einer Temperatur zwischen −30 und 100°C mit Salpetersäure als Nitrierungsmittel, man überraschenderweise nach einem einfachen, ganz allgemein für mindestens monosubstituierte Arylverbindungen anwendbares, ökologisch günstigen Nitrierverfahren die entsprechenden Nitroarylverbindungen in hoher Ausbeute erhält.

Eine geringe Sättigungslöslichkeit des Reaktionsproduktes in der organischen Phase kann durchaus vorhanden sein, da die Phase recycliert wird und somit weder eine weitere Aufkonzentrierung noch ein Produktverlust erfolgt.

Eine weitere Besonderheit des erfindungsgemässen Verfahrens ist, dass die anorganische Phase in manchen Fällen in bezug auf die Nitroverbindung in der organischen Phase extrahierend wirken kann. Aus diesem Grunde ist die Verwendung auch von solchen organischen Flüssigkeiten noch möglich, in denen das Reaktionsprodukt normalerweise mehr oder weniger löslich ist, in Gegenwart von Schwefelsäure die Produktlöslichkeit aber stark abnimmt.

Im einzelnen sind auch die jeweiligen Reaktionsbedingungen für die Möglichkeiten der Anwendung von Inertflüssigkeit und Nitrierungssubstrat von Bedeutung.

So sind Chlorbenzole bei milderen Nitrierungsbedingungen z.B. bei einer Temperatur zwischen − 10 bis 30°C und einer Schwefelsäurekonzentration der anorganischen Phase von ca. 90% noch ausgezeichnete Inertflüssigkeiten während der Nitrierung. Wendet man dagegen strenge Nitrierungsbedingungen an, z.B. bei einer Temperatur von 50°C oder darüber und Schwefelsäuremonohydrat als anorganische Phase, so können gemäss dem neuen Verfahren auch die vorher als Inertflüssigkeit eingesetzten Chlorbenzole nitriert werden, vorausgesetzt, das Phasentrennungsprinzip des Nitrierungsproduktes zwischen anorganischer Phase und organischer Inertphase bleibt erhalten. Im letzten Falle müssen natürlich solche organische Inertflüssigkeiten eingesetzt werden, die bei den strengen Nitrierungsbedingungen trotzdem reaktionsinert bleiben. Als solche gelten Benzine der verschiedenen Siedebereiche, insbesondere der Siedebereiche zwischen 130 bis 200°C.

Mindestens monosubstituierte Arylverbindungen, die gemäss dem erfindungsgemässen Verfahren zur Nitrierung geeignet sind, sind z.B. solche, die eine Alkoxygruppe oder Acylamidogruppe aufweisen, vor allem aber solche mit elektronegativen Substituenten, wie der Carboxylgruppe, der Cyangruppe oder Halogen, wie Brom oder insbesondere Chlor.

Als Beispiele seien genannt: Benzoesäure, o- und p-Chlorbenzoesäure, Acetylanilin, 4-Acetamido-1-methoxybenzol, 4-Propionamido-1-methoxybenzol, 4-Cyanacetanilid, 4-Methoxyacetanilid, 4-Methoxyäthoxyanilin, 2,5-Dichloracetanilid, 2,5-Dibromacetanilid, 2,6-Dichlorpropionanilid, 2,4-Dichlorbenzol, 2,6-Dijodanilin, 2-Chloranilin, 4-Dimethylaminotoluol.

Als für das neue Verfahren verwendbare, unter den Reaktionsbedingungen inerte organische Flüssigkeiten kommen vor allem nichtpolare organische Lösungsmittel, wie z.B. aliphatische und aromatische Chlorkohlenwasserstoffe, aliphatische Kohlenwasserstoffe u.a. in Frage, wie z.B. Chlorbenzol oder o-Dichlorbenzol, sowie Tetrachloräthan, insbesondere aber Benzine verschiedener Siedebereiche, wie z.B. Testbenzin, Lackbenzin, Naphtha usw. Die einzelnen flüssigen Kohlenwasserstoffe können natürlich auch in reiner Form eingesetzt werden, z.B. als n-Oktan, n-Heptan usw.

Es könen jedoch auch andere organische Lösungsmittel eingesetzt werden, sofern sie den definierten Parametern entsprechen.

Die Menge an inertem organischem Lösungsmittel ist in grösserem Bereich variierbar und letztlich vor allem zur besseren Rührbarkeit bzw. Pumpbarkeit und Wärmeabführung nötig. Da aber gut 95% wieder zurückgewonnen werden und ohne weitere Aufarbeitung wieder einsetzbar sind, spielt die Quantität nur im Hinblick auf die Produktionskapazität eine gewisse Rolle.

Günstig ist ein Gewichtsverhältnis von Lösungsmittel zu eingesetztem Substrat von 3 : 1 bis 8 : 1.

Die gemäss der Erfindung als anorganische Phase anzuwendende Schwefelsäure kann aus 100%iger $H_2SO_4$, sogenanntem Monohydrat ($SO_3 \cdot H_2O$) bestehen, sie kann jedoch auch eine geringere Menge Wasser enthalten. Konzentrierte Schwefelsäure von Handelsqualität, die gewöhnlich 96% $H_2SO_4$ und 4% Wasser enthält, ist anwendbar. Sie kann aber auch bis zu 10% Schwefeltrioxyd, berechnet auf das Monohydrat, enthalten. Die Schwefelsäure sollte jedoch mindestens eine $H_2SO_4$-Konzentration von 80% besitzen. Für die vielseitige praktische Anwendung ist der Bereich von 90 bis 95% $H_2SO_4$ am günstigsten.

Die Menge Schwefelsäure, berechnet als $H_2SO_4$, soll vorzugsweise zwischen 500 und 1000 Gewichtsprozent, bezogen auf das Reaktionssubstrat, betragen. In manchen Fällen reicht jedoch auch weniger, z.B. 300%, aus.

Die zur Nitrierung angewandte Salpetersäure soll eine Stärke besitzen, die gewöhnlicher konzentrierter (ca. 64%iger) Salpetersäure entspricht. Die Nitrierung wird zweckmässig mittels einer Mischung von Salpetersäure und Schwefelsäure der sogenannten Nitriersäure durchgeführt, kann jedoch auch mit Salpetersäure alleine durchgeführt werden.

Das Gewichtsverhältnis von Schwefelsäure-Monohydrat zu konz. Salpetersäure in der Mischsäure ist dabei etwa 1 : 1.

Vorzugsweise wird die Salpetersäurekonzentration in dieser Nitriersäure so eingestellt, dass sie in geringem Überschuss zur theoretisch zur Mononitrierung notwendigen Menge vorhanden ist. Der Überschuss beträgt in der Regel ca. 5%. Man kann auch konzentrierte Salpetersäure alleine als Nitriermittel einsetzen, da Schwefelsäure ohnehin als Reaktionsmedium vorliegt. Die Menge an eingesetztem Schwefelsäure-Monohydrat muss dann allerdings entsprechend erhöht werden, sonst verhält sich das übrige Säuregemisch wie eine niedrigprozentige Säure, mit der gegebenenfalls schlechtere Ausbeuten und eventuell ungünstigere Isomerenverhältnisse in bezug auf das gewünschte Isomer erhalten werden würden.

Das Verfahren wird beipielsweise wie folgt durchgeführt. Man legt die organische Inert-Flüssigkeit in etwa 3- bis 8facher Menge, bezogen auf eingesetztes Reaktionssubstrat, vor. Danach wird das Reaktionssubstrat in den Reaktor eingetragen und anschliessend etwa die sechsfache Menge, bezogen auf das Reaktionssubstrat, 85–100%ige Schwefelsäure hinzugesetzt. Das Reaktionssubstrat wird in dieser Phase durch die Schwefelsäure gelöst.

Man stellt danach auf Reaktionstemperatur und lässt daraufhin die Nitriersäure zulaufen, die mengenmässig ca. 5–10% über der stöchiometrisch notwendigen Menge zur Mononitrierung der eingesetzten Reaktionssubstrate liegt, wobei die Temperatur im gleichen Bereich gehalten wird.

Es wird anschliessend zur Nachreaktion nachgeführt, wonach die Phasentrennung erfolgt. Die anorganische Phase lässt man in kaltes Wasser laufen und verdünnt nur so weit, wie es zur Produktausfällung erforderlich ist.

Oft reicht eine Herabsetzung der Konzentration auf eine Schwefelsäurekonzentration von 60–80%.

In einem solchen Falle kann die Filtratsäure durch Aufkonzentrieren mittels $SO_3$ recycliert und im gleichen Verfahren wieder eingesetzt werden. Das Produkt wird abfiltriert, mit Wasser gewaschen und gegebenenfalls getrocknet.

Das erfindungsgemässe Verfahren schliesst eine Arbeitsweise bei Temperaturen oberhalb 50 bis 100 °C natürlich nicht aus, wodurch auch schwieriger zu nitrierende Substanzen nitriert werden können. Dies ist insbesondere wegen der vorteilhaften Wärmeabfuhr möglich.

Aufgrund der herzustellenden Produkte und deren Empfindlichkeit auf Nebenproduktbildung wird jedoch in erster Linie bei niedrigen Temperaturen zwischen −10 bis 50 °C, vorzugsweise im Temperaturbereich von 10 bis 35 °C, nitriert.

Das neue Verfahren bietet bei den einzelnen zu nitrierenden Substraten z.B. folgende Vorteile, und zwar:

a) dass unter den Bedingungen des Zweiphasenverfahrens praktisch keine Sulfonierung mehr auftritt und damit verbunden höhere Ausbeuten erhalten werden.

b) Die veränderten Reaktionsbedingungen erlauben die Durchführung der Nitrierung bei erhöhter Temperatur, ohne dass Nebenreaktionen eintreten und wodurch die Wärmeabfuhr erleichtert wird. Ein Übergang von teurem Trockeneis auf Solekühlung als Kühlmedium ist möglich.

c) Durch die wesentlich bessere Wärmeabführung ist eine schnellere Reaktionsführung möglich, was in besseren Raum-Zeit-Ausbeuten ausgedrückt werden kann.

d) Es ergeben sich erhebliche Energieeinsparungen wegen dem nur geringeren Erfordernis der Kühlung bzw. des Aufheizens gegenüber bisherigen Verfahren.

e) Ökologische Vorteile ergeben sich aus der Einsparung der Aufarbeitungsprozeduren und der Wiederverwendung der organischen Inertflüssigkeit.

Die gemäss dem neuen Verfahren hergestellten Verbindungen können wertvolle Farbstoffzwischenprodukte sein, welche beispielsweise nach Reduktion der Nitro- zur Aminogruppe als Kupplungskomponenten zur Herstellung von Azofarbstoffen oder Azopigmenten oder als Diazokomponenten bei der Azofarbstoffherstellung eingesetzt werden können. Sie stellen jedoch auch als solche in verschiedenen Bereichen der Chemie einsetzbare Chemikalien dar.

Die folgenden Beispiele veranschaulichen das neue Verfahren, ohne es auf diese zu beschränken.

Beispiel 1
Nitrierung von Benzoesäure

In einem 1,5-l-Kolben mit Untenauslauf werden 500 g (ca. 700 ml) Benzin vom Siedepunkt 110 bis 140 °C vorgelegt. Unter Rühren trägt man 122,1 g Benzoesäure 100% ein und fügt 732 g Schwefelsäure 98%ig hinzu. Die entstandene Suspension wird jetzt auf ca. 55 °C erwärmt und so lange gerührt (ca. 10 Minuten), bis die Benzoesäure mit hellgelber Farbe in der Schwefelsäure in Lösung gegangen ist. Anschliessend wird das 2-Phasengemisch wieder auf 20 bis 25 °C abgekühlt, ohne dass es hierbei zur Auskristallisation der Benzoesäure kommt. Jetzt lässt man 131,3 g Nitriersäure 50,4%ig zulaufen und hält die Temperatur mittels Kühlbad bei max. 20 bis 25 °C. Zulaufdauer ca. 30 bis 40 Minuten je nach Kühlung. Im Anschluss wird noch 30 Minuten ausgerührt. Gesamtes Volumen: ca. 1300 ml, davon: obere Phase (Benzin) ca. 700 ml, farblos, untere Phase (Reaktionsmasse) ca. 600 ml, gelbbraun.

In einem 4-l-Becherglas legt man nun ca. 1,8 l kaltes Wasser vor und lässt die untere Phase des Reaktionsgemisches innerhalb von ca. 20 bis 30 Minuten unter Rühren zulaufen. Die Temperatur im Becherglas steigt dabei bis auf 55 bis 60 °C an. Durch Wasser- bzw. Eiszugabe hält man die Temperatur in diesem Bereich konstant. Das Endvolumen wird danach auf 3 l gestellt, dann wird noch 30 Minuten bei 55 bis 60 °C nachgerührt.

Mittels Eisbad kühlt man die beigefarbene Suspension auf 40 °C ab und rührt nochmals 30 Minuten nach, um stationäre Verhältnisse zu erhalten. Die Filtration erfolgt bei gleicher Temperatur von 40 °C über eine Nutsche von 16 cm ∅: Mit ca. 1 l kaltem Wasser wird in Portionen nachgewaschen. Der Ablauf hat gegen Ende des Auswaschens einen pH-Wert von ca. 2,5 bis 2,6. Die Filtrierzeit beträgt ca. 30 Sekunden, die Waschzeit ca. 45 Sekunden.

Die Trocknung des Produktes erfolgt bei 70 bis 80 °C unter Wasserstrahlvakuum. Man erhält m-Nitrobenzoesäure mit einem Schmelzpunkt von 140 °C (Literaturangaben 140 bis 141 °C). Der Gehalt an Reinprodukt nach LC-Analyse beträgt 98,5 bis 99,5%. Ausbeute: ca. 128 g t.q. zu ca. 99% (nach LC) = ca. 127 g zu 100%, was 76% der Theorie ausmacht.

Im Scheidekolben verbleibt ein Rest von ca. 490 g Benzin, das ist ca. 98% des Einsatzes, der beim nächsten Ansatz wieder verwendet werden kann.

Beispiel 2
Nitrierung von 2',5'-Dichloracetanilid

In einem 2,5-l-Kolben werden 550 g Benzin Kp. 110–140 °C vorgelegt. Dann trägt man unter Rühren 162,0 g 2,5-Dichloranilin zu 100% ein und erwärmt auf 45 °C, wobei Lösung eintritt. Innerhalb von ca. 15 Minuten lässt man darauf 102 g Acetanhydrid 100%ig zutropfen und lässt die Temperatur bis auf 65 °C ansteigen. Nachdem 15 Minuten bei 65 °C gerührt wurde, beginnt man mit dem Zulauf von 1265 g 98%iger Schwefelsäure und achtet darauf, dass die Temperatur nicht wesentlich über 70–75 °C steigt. Nachdem ca. 20–25% der Schwefelsäure zugelaufen sind, beginnt sich das vorher ausgefallene Acetanilid zu lösen. Der Zulauf der restlichen Schwefelsäure ist dann kaum mehr exotherm, und die Temperatur sinkt auf ca. 60 °C ab. Durch weitere Kühlung bringt man das 2-Phasengemisch auf 20–25 °C und beginnt sofort bei max. 25 °C mit dem Zulauf von 128,2 g Nitriersäure zu 50,2%. Nach etwa 50 Minuten ist der Zulauf beendet, und man rührt noch 20 Minuten bei 20 bis 25 °C aus.

Das Reaktionsgemisch wird nun in einen 2-l-Scheidetrichter gebracht, worauf sich sofort eine untere, dunkelbraune Schicht abscheidet. Die obere Benzin-Schicht ist zumeist farblos oder nur schwach gelb gefärbt. Man lässt jetzt die untere Schicht langsam in ein Becherglas fliessen, in dem ein Liter Wasser von 40 °C gerührt wird. Durch dauernde Zugabe von insgesamt ca. 1300 g Eis hält man die Temperatur während der Dauer des Zulaufs von ca. 30 Minuten auf max. 50 °C. Am Ende resultiert eine braungelbe Suspension. Man rührt einige Minuten bei 50 bis 55 °C aus und filtriert ab.

Mit 1,5 l kaltem Wasser wird das Nutschgut gewaschen. Falls das Filtrat noch nicht neutral ist, wird nachgewaschen, gut abgepresst und gegebenenfalls getrocknet. Mutterlauge und Waschwasser werden vereinigt. Die im Scheidetrichter verbliebene Menge Benzin beträgt ca. 536 g und kann nach Ergänzung der fehlenden Menge tel quel beim nächsten Ansatz wieder verwendet werden. Ausbeute ca. 235 g 2',5'-Dichlor-4'-nitroacetanilid. Das nach dieser Methode erhaltene Produkt ist genügend rein, dass es in der Farbstoff- bzw. Pigmentherstellung direkt eingesetzt werden kann.

Beispiel 3
Nitrierung von 4'-Methoxyacetanilid

In einem 2,5-l-Kolben werden 800 g Chlorbenzol vorgelegt. Dann trägt man unter Rühren 123,2 g p-Anisidin zu 100% am besten als Schmelze ein. Innerhalb von ca. 15 Minuten lässt man darauf 102 g Acetanhydrid zutropfen, wobei die Temperatur bis auf 65 °C ansteigt. Nachdem 15 Minuten bei 65 °C angerührt wurde, kühlt man innerhalb von etwa 10 Minuten mit Hilfe eines Kühlbades auf ca. 30 °C ab.

Dabei beginnt das Acetanisidid im Bereich von ca. 45 °C in beigefarbenen Kristallen auszufallen.

Die erhaltene Suspension ist leicht viskos, aber gut rührbar.

Nun beginnt man mit dem Zulauf von 800 g 92%iger Schwefelsäure und achtet darauf, dass die Temperatur nicht weit über 30–40 °C ansteigt. Dauer des Zulaufs ca. 10 Minuten. Unter weiterer Kühlung beginnt man bei max. 25 °C sofort mit dem Zulauf von 101,2 g konz. Salpetersäure zu 62,3%. Dabei kann man die Temperatur bis auf 20 bis 25 °C ansteigen lassen. Mit den ersten Tropfen $HNO_3$ beginnt die Emulsion sofort dünnflüssig zu werden, und die vorher blaue Farbe schlägt nach braun um. Nach etwa 10 Minuten ist der Zulauf beendet, und man rührt noch 20 Minuten bei 20 bis 25 °C. Volumen der Lösung ca. 1450 ml.

Das Reaktionsgemisch wird nun in einen 2-l-Scheidetrichter gebracht, worauf sich sofort eine untere, dunkelbraune Schicht abscheidet. Die obere, chlorbenzolische Schicht ist zumeist farblos oder nur schwach gelb gefärbt. Man lässt jetzt die untere Schicht langsam in ein Becherglas fliessen, in dem ein Gemisch von 300 g Eis/Wasser gerührt wird. Durch dauernde Zugabe von insgesamt ca. 1500 g Eis hält man die Temperatur um +5 °C. Dauer des Zulaufs ca. 30 Minuten. Am Ende resultiert eine dicke, gelbe Suspension. Man rührt einige Minuten aus und filtriert ab.

Mit 350 ml Eiswasser wird nachgewaschen und gut abgepresst und gegebenenfalls getrocknet. Mutterlauge und Waschwasser werden vereinigt. Die im Scheidetrichter verbliebene Menge Chlorbenzol beträgt ca. 760 g und kann nach Ergänzung der fehlenden Mengen tel quel beim nächsten Ansatz wieder verwendet werden. Ausbeute ca. 193 bis 95 g 4-Acetamido-2-nitroanisol mit 97–98%iger Reinheit. Schmelzpunkt ca. 150 bis 151 °C.

Das nach dieser Methode erhaltene Produkt ist genügend rein, um in einer nächsten Synthesestufe zu 3′-Benzylamino-4′-methoxyacetanilid umgesetzt zu werden.

## Patentansprüche

1. Verfahren zur Herstellung von mindestens monosubstituierten Nitroarylverbindungen durch chargenweise Nitrierung von mindestens monosubstituierten Arylverbindungen, dadurch gekennzeichnet, dass man die Nitrierung in einem Zweiphasensystem, bestehend aus einer anorganische Phase aus mindestens 80%iger Schwefelsäure und einer organischen Phase, bestehend aus einer reaktionsinerten organischen Flüssigkeit, in der das Nitrierungsprodukt in Gegenwart von Schwefelsäure praktisch unlöslich ist, bei einer Temperatur zwischen −30 bis 100 °C mit Salpetersäure als Nitrierungsmittel durchführt.

2. Verfahren gemäss Anspruch 1, dadurch gekennzeichnet, dass man als reaktionsinerte organische Flüssigkeiten nichtpolare organische Lösungsmittel verwendet.

3. Verfahren gemäss Anspruch 3, dadurch gekennzeichnet, dass man als reaktionsinerte organische Flüssigkeiten aliphatische und aromatische Chlorkohlenwasserstoffe sowie aliphatische Kohlenwasserstoffe verwendet.

4. Verfahren gemäss Anspruch 3, dadurch gekennzeichnet, dass man als reaktionsinerte organische Flüssigkeiten Chlorbenzol, o-Dichlorbenzol, Tetrachloräthan, Benzine verschiedener Siedebereiche, wie z.B. Testbenzin, Lackbenzin, Naphtha, verwendet.

5. Verfahren gemäss Anspruch 4, dadurch gekennzeichnet, dass man als reaktionsinerte organische Flüssigkeit Benzine vom Siedebereich 130 bis 200 °C verwendet.

6. Verfahren gemäss Anspruch 1, dadurch gekennzeichnet, dass man als anorganische Phase 85- bis 100%ige Schwefelsäure verwendet.

7. Verfahren gemäss Anspruch 1, dadurch gekennzeichnet, dass man die Schwefelsäure, berechnet als $H_2SO_4$, in einer Menge von 300–1000 Gewichtsprozent, bezogen auf die Menge des Reaktionssubstrates, einsetzt.

8. Verfahren gemäss Anspruch 1, dadurch gekennzeichnet, dass man die Nitrierung mit einem Gemisch von konzentrierter Schwefelsäure (Monohydrat) und konzentrierter Salpetersäure im Gewichtsverhältnis von 1 : 1 der sogenannten Nitriersäure durchführt.

9. Verfahren gemäss Anspruch 1, dadurch gekennzeichnet, dass man die Nitrierung bei einer Temperatur zwischen 10 bis 35 °C durchführt.

## Claims

1. A process for the production of nitroaryl compounds which are at least monosubstituted, by batchwise nitration of aryl compounds which are at least monosubstituted, which process comprises carrying out the nitration in a two-phase system which consists of an inorganic phase consisting of sulfuric acid having a concentration of at least 80% and an organic phase consisting of an inert organic liquid in which the nitration product is almost insoluble in the presence of sulfuric acid, at a temperature in the range from −30 to 100 °C, with nitric acid as nitrating agent.

2. A process according to claim 1, wherein the inert organic liquid is a nonpolar organic solvent.

3. A process according to claim 1, wherein the inert organic liquid is an aliphatic or aromatic chlorinated hydrocarbon, or an aliphatic hydrocarbon.

4. A process according to claim 3, wherein the inert organic liquid is chlorobenzene, o-dichlorobenzene, tetrachloroethane, a petroleum distillate of different boiling range, for example white spirit or naphtha.

5. A process according to claim 4, wherein the inert organic liquid is a petroleum spirit with a boiling range from 130 to 200 °C.

6. A process according to claim 1, wherein the inorganic phase is sulfuric acid having a concentration of 85 to 100%.

7. A process according to claim 1, wherein the sulfuric acid, calculated as $H_2SO_4$, is employed in an amount of 300 to 1000% by weight, based on the amount of the reaction substrate.

8. A process according to claim 1, wherein the nitration is carried out with a mixture of concentrated sulfuric acid (monohydrate) and concentrated nitric acid in the weight ratio of 1 : 1.

9. A process according to claim 1, wherein the nitration is carried out at a temperature in the range from 10 to 35°C.

**Revendications**

1. Procédé de préparation de composés nitroaryliques au moins monosubstitués par nitration discontinue de composés aryliques au moins monosubstitués, caractérisé par le fait qu'on effectue la nitration dans un système à deux phases comprenant une phase minérale constituée par au moins de l'acide sulfurique à 80% et une phase organique constituée par au moins un liquide organique inerte vis-à-vis de la réaction, dans lequel le produit de nitration est pratiquement insoluble en présence d'acide sulfurique, à une température comprise entre −30 et 100°C, avec l'acide nitrique comme agent nitrant.

2. Procédé selon la revendication 1, caractérisé par le fait qu'on utilise comme liquide organique inerte vis-à-vis de la réaction des solvants organiques non polaires.

3. Procédé selon la revendication 2, caractérisé par le fait qu'on utilise comme liquide organique inerte vis-à-vis de la réaction des hydrocarbures chlorés aliphatiques et aromatiques ainsi que des hydrocarbures aliphatiques.

4. Procédé selon la revendication 3, caractérisé par le fait qu'on utilise comme liquide organique inerte vis-à-vis de la réaction le chlorobenzène, l'o-dichlorobenzène, le tétrachloréthane, des essences à plages d'ébullition différentes telles que le white spirit, l'essence pour vernis, le naphta.

5. Procédé selon la revendication 4, caractérisé par le fait qu'on utilise comme liquide organique inerte vis-à-vis de la réaction des essences ayant des plages d'ébullition comprises entre 130 et 200°C.

6. Procédé selon la revendication 1, caractérisé par le fait qu'on utilise comme phase minérale de l'acide sulfurique à 85–100%.

7. Procédé selon la revendication 1, caractérisé par le fait qu'on utilise l'acide sulfurique, calculé en $H_2SO_4$, en une quantité comprise entre 300 et 1000 pour cent en poids par rapport à la quantité du substrat réactionnel.

8. Procédé selon la revendication 1, caractérisé par le fait qu'on effectue la nitration avec un mélange d'acide sulfurique concentré (monohydrate) et d'acide nitrique concentré dans un rapport pondéral de 1 : 1, appelé mélange sulfonitrique.

9. Procédé selon la revendication 1, caractérisé par le fait qu'on effectue la nitration à une température comprise entre 10 et 35°C.